# EUROPEAN PATENT SPECIFICATION

(11) **EP 2 526 907 B1**
(45) Date of publication and mention of the grant of the patent: **23.10.2013**
(21) Application number: 11004231.4
(22) Date of filing: 23.05.2011
(51) Int. Cl.: A61F 5/44

(54) **Urine meter**
Urinmessgerät
Mesureur d'urine

(43) Date of publication of application: 28.11.2012
(73) Proprietor: Covidien LP, Mansfield, MA 02048 (US)
(72) Inventor: Fitzgerald, Alan, Edgeworthtown, Co. Longford (IE); Deighan, Ciara, Birr, Co. Offaly (IE); Daly, Paul J., Tullamore, Co. Offaly (IE); Robinson, Tom, Roscrea, Co. Tipperary (IE)
(74) Representative: Gray, James

(56) References cited:
- EP-A1- 0 471 413
- EP-A2- 0 983 746
- EP-A2- 1 825 836
- US-A- 4 000 649
- US-A- 4 095 589

## Description

The present invention relates to a urine meter, and in particular to a urine meter for monitoring the flow of urine from a catheterised patient.

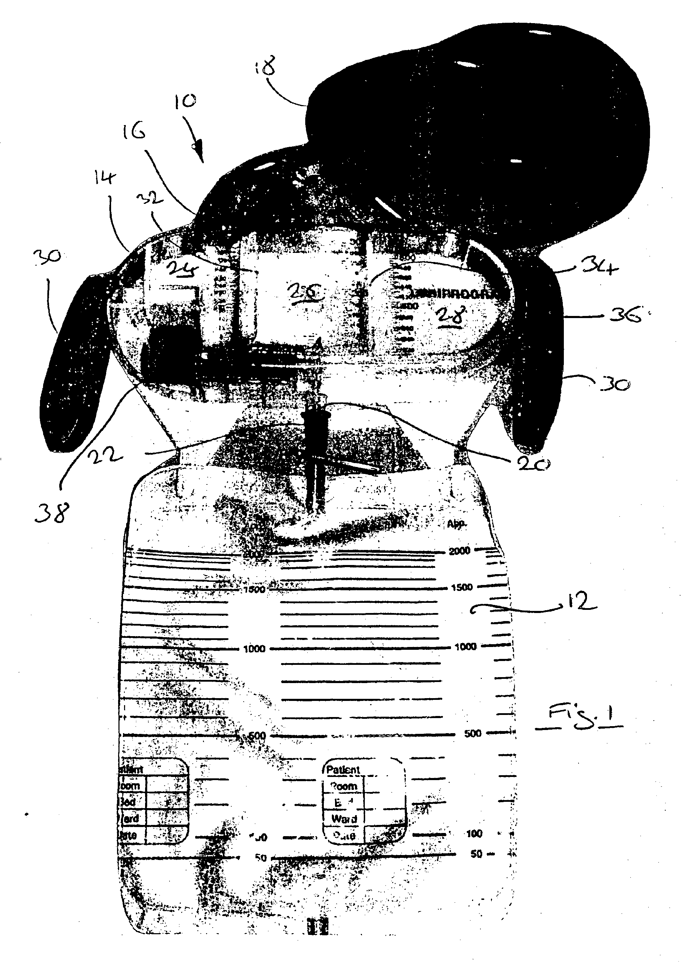

Figure 1 shows a known urine meter, generally designated 10, and a urine collection bag 12. The urine meter 10 includes a housing 14 that is provided with an inlet 16 having an inlet conduit 18. The inlet conduit is in use, connectable to a urinary catheter of a patient. The housing 14 is further provided with an outlet 20 which is connected by an outlet conduit 22 to the collection bag 12. Straps 30 are provided on the urine meter 10 to permit both the meter 10 and collection bag 12 to be mounted to an appropriate support. The support may, for example, be the rail of a patient bed.

The housing 14 further defines first, second and third measurement chambers 24,26,28. For the urine meter 10 shown, the first measurement chamber 24 has a volume of 100 millilitres, the second measurement chamber 26 has a volume of 200 millilitres, and the third measurement chamber 28 has a volume of 200 millilitres. The housing 14 is formed from a clear plastic material such as, for example, a styrenebutadiene copolymer. One such suitable material is K-Resin^{RTM} SBC. The interior of each measurement chamber 24,26,28 is viewable through the housing 14, and each chamber 24,26,28 is marked with graduations which permit the volume of liquid in each chamber 24,26,28 to be measured. The first chamber 24 is marked with graduations from 0ml to 100ml, the second chamber 26 is marked with graduations from 100 ml to 300ml, and the third chamber is marked with graduations from 300ml to 500ml. The first chamber 24 is separated from the second chamber 26 by an interior wall 32 of the housing 14 which defines a weir between the chambers 24,26. The second chamber 26 is similarly separated from the third chamber 28 by a further interior wall 34 of the housing 14 which defines a weir between the chambers 26,28.

The housing 14 is configured such that liquid entering the meter 10 through the inlet 16 flows firstly into the first chamber 24. Once the first chamber 24 has been filled, incoming liquid spills over the weir defined by the wall 32 separating the first and second chambers 24,26 and into the second chamber 26. Once the second chamber 26 has been filled, incoming liquid spills over the weir defined by the wall 34 separating the second and third chambers 26,28 and into the third chamber 28. The urine meter 10 is further provided with an overflow conduit 36 which connects the third chamber 28 to the outlet 20. In the event that the first, second and third chambers 24,26,28 become filled, excess liquid is conducted through the overflow conduit 36 to the outlet 20.

The meter 10 is further provided with a drainage tap 38 which is located in a lower region of the housing 14. The drainage tap 38 enables the chambers 24,26,28 to be placed in fluid communication with the outlet 20 and thereby permit liquid present in the chambers 24,26,28 to be drained to the collection bag 12. The drainage tap 38 is configured so as to permit the sequential drainage of the chambers 24,26,28.

One drawback of the urine meter 10 of figure 1 is that in the event of the meter 10 being tipped or shaken, for example when the meter 10 is moved, liquid can transfer between the chambers 24,26,28 by passing over the chamber separating walls 32,34. This can thus lead to the meter 10 providing an incorrect reading.

Although not particularly relevant to the present invention, urine meters are described in EP0471413A1, EP1825836A2, US4000649A, US4095589A, and EP0983746A2.

According to the present invention there is provided a urine meter having a housing which includes an inlet and an outlet, the housing further including a first measurement chamber and a second measurement chamber arranged such that liquid entering the inlet enters the first measurement chamber before passing to the second measurement chamber, wherein the housing is further provided with a non-return valve between the first and second measurement chambers which, in use, prevents the flow of liquid from the second measurement chamber to the first measurement chamber.

The non return valve may be biased to an open condition to permit fluid communication between the first chamber and the second chamber, and urged to a closed condition to prevent fluid communication between the chambers by the action of liquid present within the second chamber. The non return valve may be biased to the open condition by gravity. The non return valve may be urged to the closed condition by the impact of liquid contained in second chamber. Such impact may occur as a result of sloshing of the liquid within the second chamber resulting from movement of the meter. The non return valve may be urged to the closed condition due to the buoyancy of a portion of the non return valve. The non return valve may be urged to the closed condition by the rising free surface of the liquid.

The non return valve is provided in an internal partition of the housing which separates the first chamber from the second chamber. The non return valve may include a valve member which is mounted to the internal partition, and at least one aperture which extends through the internal partition and which defines fluid communication channel between the first and second measurement chambers. In such an embodiment the valve member may include a stem and a head, wherein the head defines a sealing surface which is movable into contact with the internal partition to close the at least one aperture. The sealing surface many be an annular sealing surface.

The stem of the valve member may received in an aperture of the internal partition. The valve member may be retained in the aperture of the internal partition by the head of the valve member and a formation on the stem of the valve member. The formation on the stem of the valve member is resiliently deformable so as to enable the valve member to be fitted to the aperture of the internal partition. The internal partition is provided with a plurality of apertures which extend through the internal partition and which define fluid communication channels between the first and second measurement chambers.

In an alternative embodiment, the housing including a third measurement chamber which, in use receives liquid from the second measurement chamber, wherein the housing is provided with a further non-return valve between the second and third measurement chambers which, in use, prevents the flow of liquid from the third measurement chamber to the second measurement chamber. In such an embodiment it will be appreciated that the non return valve may have the same configuration and operating characteristics as non return valve described with reference to the previously described two chamber embodiment.

In such an embodiment the further non-return valve is preferably positioned lower in the housing than the first non-return valve. This ensures liquid is able to flow from the second measurement chamber to the third measurement chamber before closure of the non-return valve provided between the first and second measurement chambers.

An embodiment of the present invention will now be described with reference to the accompanying drawings in which:
Figure 1 shows a known urine meter and collection bag;
Figure 2 shows a cross-sectional view of a urine meter;
Figure 3 shows a cross-sectional view of a non-return valve of the urine meter of figure 2, the valve being in an open position;
Figure 4 shows a cross-sectional view of the non-return valve of the urine meter of figure 2, the non-return valve being in a closed position;
Figure 5 shows a perspective view of the non-return valve;
Figure 6 shows a perspective view of the valve member; and
Figure 7 shows a perspective view of the non-return valve with the valve member removed.

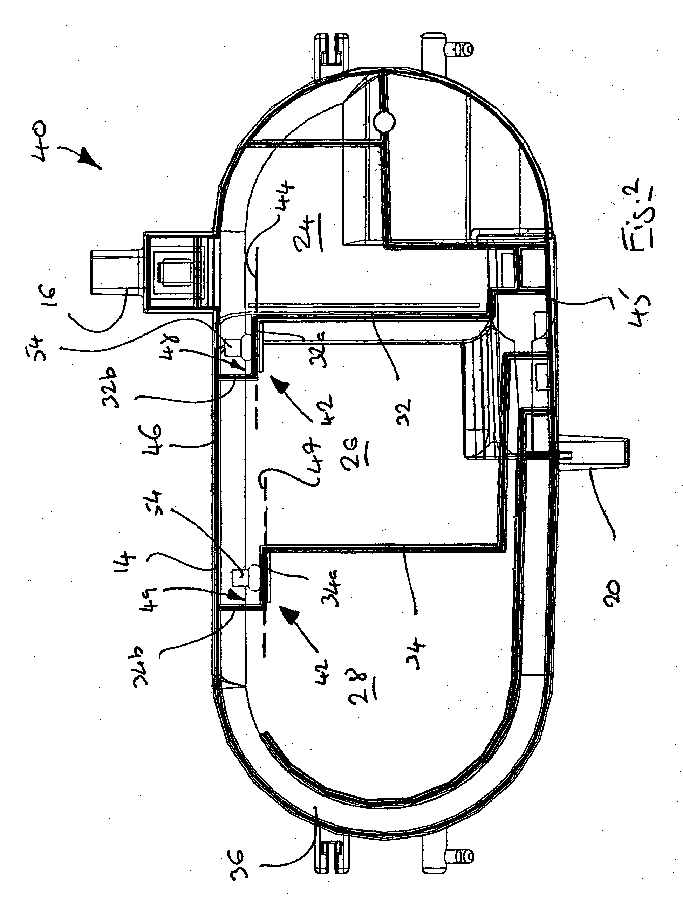

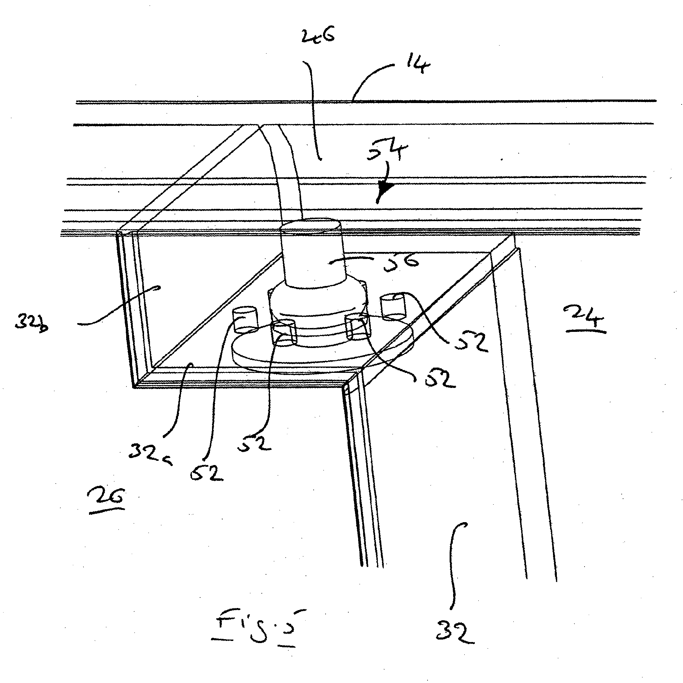

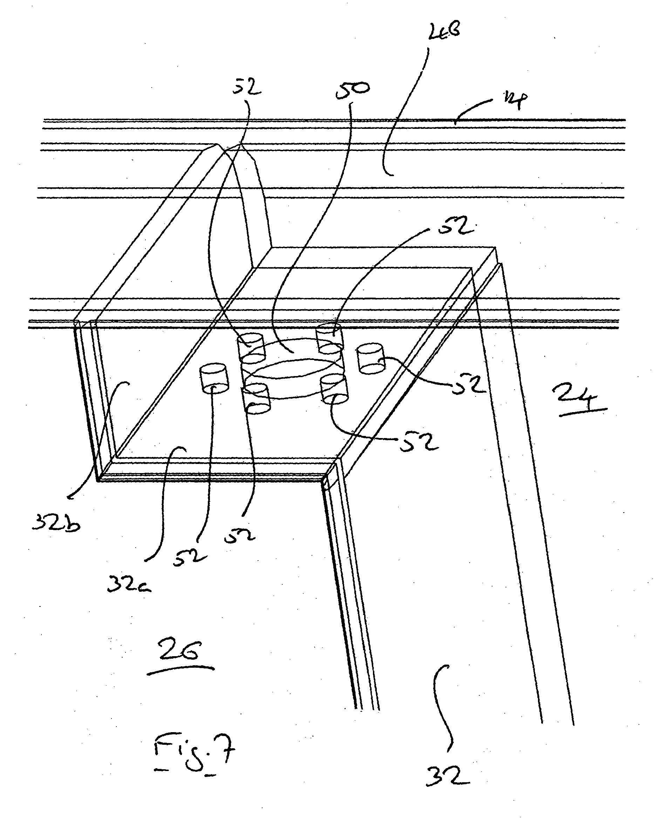

Referring now to figures 2 to 7 there is shown a urine meter, generally designated 40. Features common to the urine meter 10 described with reference to figure 1 are identified with like reference numerals.

Figure 2 shows a cross-sectional view of the housing 14 of the urine meter 40 viewed from the opposite side to that shown in figure 1. The housing 14 of the meter 40 differs from that described with reference to figure 1 in that non return valves, generally designated 42, are provided between the first and second chambers 24,26, and the second and third chambers 26,28 respectively. Each non return valve 42 is provided in an extension of the wall 32, 34 that separates the first and second chambers 24,26, and the second and third chambers 26,28 respectively.

Looking firstly at the wall 32 which separates the first and second chambers 24,26, the upper edge of the wall 32 in the meter 10 of figure 1 is indicated by broken line 44. In the meter 40 of the present invention, the wall 32 is extended such that it extends fully across the housing 14 from the base wall 45 to the top wall 46 thereof. The wall 32 is extended by an extension generally designated 48. The extension 48 includes a first portion 32a which lies on a plane substantially perpendicular to the plane of the major portion of the wall 32. The wall 32 is further extended by a second portion 32b which lies on a plane substantially perpendicular to that of the first portion 32a, and substantially parallel to the plane of the major portion of the wall 32. The first and second portions 32a,32b of the wall extension 48 thus define a step in the wall 32.

The first portion 32a of the wall extension 48 is provided with an aperture 50 configured to receive a non return valve 42. Aperture 50 may have any cross sectional area which corresponds to a cross sectional area of a portion of the return valve received within aperture 50. Wall extension 48 further comprises at least one second aperture 52 positioned in relation to aperture 50 so that when non return valve 42 is in the open position, fluid may flow through the at least one aperture 52, and when non return valve 42 is in the closed position, fluid is prevented from flowing through the at least one aperture. The at least one second aperture may, but need not have a cross sectional area less than the cross sectional area of aperture 50. In one embodiment of the invention, the wall extension 48 is provided with a substantially circular first aperture 50 and a plurality of substantially circular second apertures 52. In the embodiment shown, the first aperture 50 is provided a substantially central position on the first portion 32a of the wall extension 48. In the embodiment shown, the wall extension 48 is further provided with six second apertures 52 which are equidistantly spaced in a circular arrangement around the first aperture 50. The second apertures 52 have a diameter that is less than that of the first aperture 50. It will be appreciated that the extension 48 may be provided with a greater or lesser number of second apertures 52. As will be described in greater detail below, the first aperture 50 serves to locate and retain a valve member 54 of a non return valve 42, while the second apertures 52, in conjunction with the valve member 54, define fluid conduits extending through the wall 32.

A similar wall extension 49 is provided in the wall 34 that separates the second and third chambers 26,28. This wall extension 49 similarly includes a first portion 34a which lies on a plane substantially perpendicular to the plane of the major portion of the wall 34. The wall 34 is further extended by a second portion 34b which lies on a plane substantially perpendicular to that of the first portion 34a, and substantially parallel to the plane of the major portion of the wall 34. The wall extension 49 is provided with a similar array of apertures having a first aperture surrounded by a plurality of smaller second apertures. The upper edge of the wall 34 that separates the second and third chambers is indicated by broken line 47. The upper edge of the wall 34 is spaced a greater distance from the top wall 46 of the housing 14 than the upper edge of the wall 32 separating the first and second chambers 24,26. This results in the non-return valve 42 provided between the first and second chambers 24,26 being positioned higher in the housing 14 than the non-return valve 42 provided between the second and third chambers 26,28.

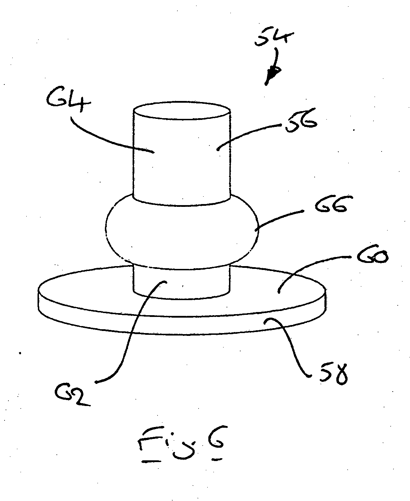

Figure 6 shows a valve member, generally designated 54, of a non return valve 42. The valve member 54 includes a stem 56 and a head 58. The head 58 of the valve member 54 may have any size and shape suitable to cover all second apertures when the non return valve 42 is in the closed position. In one embodiment, the head 58 of the valve member 54 is substantially disc shaped and is provided with an annular and substantially flat sealing surface 60. The stem 56 extends from a substantially central position on the head 58 such that the annular sealing surface 60 extends around the location at which the stem 56 meets the head 58. The opposite side of the head 58 is provided with a substantially circular face 61. The stem 56 has a first generally cylindrical portion 62, a second generally cylindrical portion 64 and a bulbous portion 66 provided between the first and second generally cylindrical portions 62,64. The diameter of the first cylindrical portion 62 is substantially equal to the diameter of the second cylindrical portion 64. The diameter of the first and second cylindrical portions 62,64 is less than the diameter of the first aperture 50 of a wall extension 48 of the housing 14. The bulbous portion 66 has an at rest diameter at its largest cross sectional area that is greater than that of the cylindrical portions 62,64 and the first aperture 50. The bulbous portion 66 is resiliently deformable so that its diameter can be reduced by compression or elongation. The valve member 54 is manufactured from a flexible plastics material.

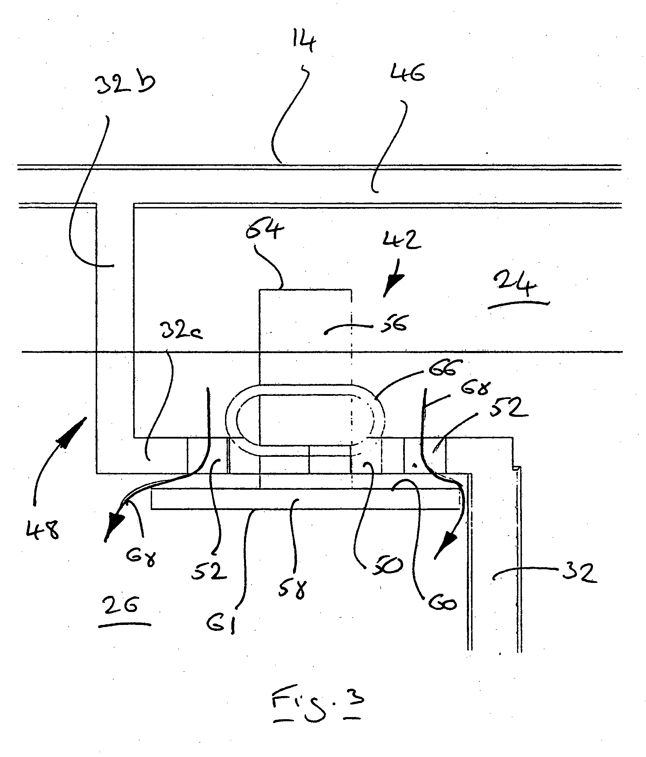

Figures 3,4 and 6 show the valve member 54 fitted to the extension 48 of a wall 32 of the housing 14. The valve member 54 is fitted to the extension 48 by first inserting the second cylindrical portion 64 of the stem 56 into the first aperture 50 of the extension 48 from the side of the extension 48 that faces the downstream chamber 26 of the meter 40. The stem 56 is inserted further into the aperture 50 such that the bulbous portion 66 deforms and passes through the aperture 50. Once clear of the aperture 50 the bulbous portion 66 returns to its original shape. It will thus be appreciated that the valve member 54 is thus retained in the aperture 50 by the presence of the bulbous portion 66 of the stem 56 on one side of the aperture 50, and the head 58 of the valve member 54 on the other side of the aperture 50. The outer diameter of the bulbous portion 66 which contacts a first surface of wall extension 48 is smaller than an inner cross sectional area of a periphery defined by each innermost point of each of the at least one second aperture so that the bulbous portion does not interfere with flow through the at least one of the second aperture. The cross sectional areal of the sealing surface 60 is greater than an outer cross sectional area of a periphery defined by each outermost point of each of the at least one second aperture so that the sealing surface 60 interrupts flow through the at least one of the second aperture when the sealing surface contacts a second surface of wall extension 48. In one embodiment, the outer diameter of the sealing surface 60 of the head 58 is greater that the outer diameter of the circular arrangement of the second apertures 52.

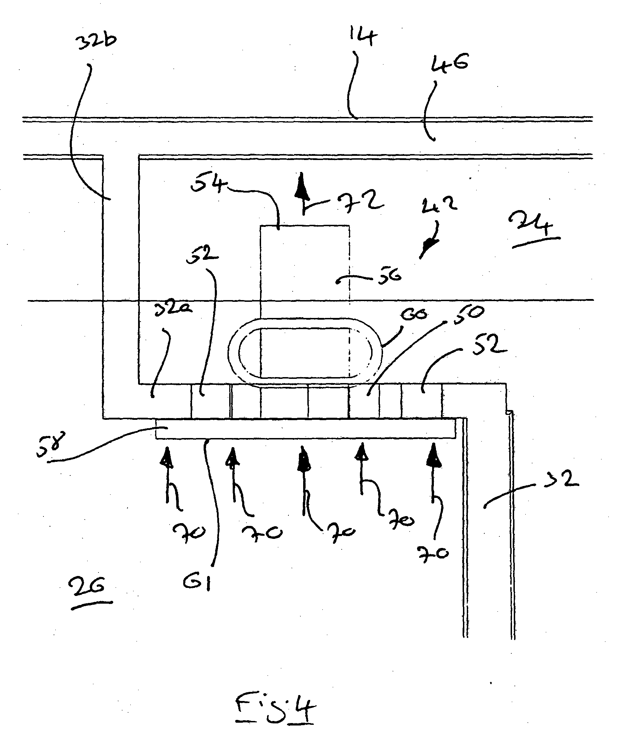

The length of the first cylindrical portion 62 of the stem 56 is greater than the thickness of the portion 32a of the wall extension 48 in which the first aperture 50 is provided. It will thus be understood that the valve member 54 is able to move between an open position, shown in figure 3, where the bulbous member 66 abuts the first aperture and the sealing surface 60 is spaced from the wall extension 48, and a closed position, shown in figure 4, where the sealing surface 60 abuts the wall extension 40 and the bulbous member 66 is spaced from the first aperture 50.

In use, the valve member 54 is gravity biased towards the open position shown in figure 3. In the first position, the arrangement of second apertures 52 are open and each second aperture 52 defines a fluid communication path through the wall extension 48. Liquid is this able to pass from the upstream chamber 24 to the downstream chamber 26 as indicated by arrows 68. In the event that liquid attempts to flow back from the downstream chamber 26 to the to the upstream chamber the liquid contacts the circular face 61 of the head 58 as indicated by arrows 70 and urges the valve member 54 to the closed position as indicted by arrow 72. The sealing surface 60 abuts a second surface of the wall extension 48 and thus closes the second apertures 52. Liquid may come into contact with the circular face 61 as a result of the downstream chamber 26 filling to the level of the first portion 32a of the wall extension 48. In such an instance, the valve member 54 is moved to the closed position due to buoyancy of the valve member 54 in the liquid. Alternatively, in instances where the downstream chamber 26 is only partially filled, the valve member 54 may be urged to the closed position as a result of sloshing of the liquid contained in the downstream chamber 26.

As described above, the non return valve 42 provided between the first and second chambers 24,26 is positioned at a higher level within the housing 14 that the non return valve 42 provided between the second and third chambers 26,28. During normal use of the urine meter 40, i.e. when it is positioned level and stably mounted to, for example, a bed frame, the differing non-return valve heights permit the chambers 24,26,28 to fill sequentially. The upper edge of the wall 34 separating the second and third chambers 26,28 is provided below the level adopted by the circular face 61 of the non return valve 42 between the first and second chambers 24,26 in the open position. As such, it will be appreciated that upon filling of the second chamber 26 to the level of the top of the wall 34 separating the second and third chambers 26,28, further liquid introduced into the second chamber 26 will flow into the third chamber 28.

In the embodiment of the invention described above, the urine meter 40 is provided with three measurement chambers 24,26,28. It will be appreciated that non-return valves 42 may be provided in urine meters having two measurement chambers or more than three measurement chambers.

## Claims

1. A urine meter (40) having a housing (14) which includes an inlet (16) and an outlet (20), the housing (14) further including a first measurement chamber (24) and a second measurement chamber (26) arranged such that liquid entering the inlet (16) enters the first measurement chamber (24) before passing to the second measurement chamber (26), **characterised in that** the housing (14) is further provided with a non-return valve (42) between the first and second measurement chambers (24,26) which, in use, prevents the flow of liquid from the second measurement chamber (26) to the first measurement chamber (24).

2. A urine meter (40) as claimed in claim 1 wherein the non return valve (42) is biased to an open condition to permit fluid communication between the first chamber (24) and the second chamber (26), and is urged to a closed condition to prevent fluid communication between the chambers (24,26) by the action of liquid present within the second chamber (26).

3. A urine meter (40) as claimed in claim 2 wherein the non return valve (42) is biased to the open condition by gravity.

4. A urine meter (40) as claimed in any preceding claim wherein the non return valve (42) is provided in an internal partition (32,32a,32b) of the housing (14) which separates the first chamber (24) from the second chamber (26).

5. A urine meter (40) as claimed in claim 4, wherein the non return valve (42) includes a valve member (54) which is mounted to the internal partition (32a), and at least one aperture (52) which extends through the internal partition (32a) and which defines fluid communication channel between the first and second measurement chambers (24,26).

6. A urine meter (40) as claimed in claim 5 wherein the valve member (54) includes a stem (56) and a head (58), wherein the head (58) defines a sealing surface (60) which is movable into contact with the internal partition (32a) to close the at least one aperture (52).

7. A urine meter (40) as claimed in claim 6 wherein the sealing surface (60) is an annular sealing surface. ,

8. A urine meter (40) as claimed in claim 6 or claim 7 wherein the stem (56) of the valve member (54) is received in an aperture (50) of the internal partition (32a).

9. A urine meter (40) as claimed in claim 8 wherein the valve member (54) is retained in the aperture (50) of the internal partition (32a) by the head (58) of the valve member (54) and a formation (66) on the stem (56) of the valve member (54).

10. A urine meter (40) as claimed in claim 9 wherein the formation (66) on the stem (56) of the valve member (54) is resiliently deformable so as to enable the valve member (54) to be fitted to the aperture (50) of the internal partition (32a).

11. A urine meter (40) as claimed in any of claims 5 to 10 wherein the internal partition (32a) is provided with a plurality of apertures (52) which extend through the internal partition (32a) and which define fluid communication channels between the first and second measurement chambers (24,26).

12. A urine meter (40) as claimed in any preceding claim, the housing (14) including a third measurement chamber (28) which, in use, receives liquid from the second measurement chamber (26), wherein the housing (14) is provided with a further non-return valve (42) between the second and third measurement chambers (26,28) which, in use, prevents the flow of liquid from the third measurement chamber (28) to the second measurement chamber (26).

13. A urine meter (40) as claimed in claim 12 wherein the further non-return valve (42) provided between the second and third measurement chambers (26,28) is positioned lower in the housing (14) than the non-return valve (42) provided between the first and second measurement chambers (24,26).

## Patentansprüche

1. Urinmessgerät (40) mit einem Gehäuse (14), das einen Einlass (16) und einen Auslass (20) aufweist, wobei das Gehäuse (14) ferner eine erste Messkammer (24) und eine zweite Messkammer (26) aufweist, die so angeordnet sind, dass in den Einlass (16) eintretende Flüssigkeit in die erste Messkammer (24) eintritt, bevor sie zu der zweiten Messkammer (26) fließt, **dadurch gekennzeichnet, dass** das Gehäuse (14) ferner mit einem Rückschlagventil (42) zwischen der ersten und der zweiten Messkammer (24, 26) versehen ist, das im Gebrauch verhindert, dass Flüssigkeit von der zweiten Messkammer (26) zu der ersten Messkammer (24) fließt.

2. Urinmessgerät (40) nach Anspruch 1, wobei das Rückschlagventil (42) zu einem offenen Zustand vorgespannt ist, um eine Fluidverbindung zwischen der ersten Kammer (24) und der zweiten Kammer (26) zu gestatten, und durch die Wirkung von in der zweiten Kammer (26) vorhandener Flüssigkeit in einen geschlossenen Zustand gedrängt wird, um eine Fluidverbindung zwischen den Kammern (24, 26) zu verhindern.

3. Urinmessgerät (40) nach Anspruch 2, wobei das Rückschlagventil (42) durch Schwerkraft zu dem offenen Zustand vorgespannt ist.

4. Urinmessgerät (40) nach einem der vorhergehenden Ansprüche, wobei das Rückschlagventil (42) in einer inneren Trennwand (32, 32a, 32b) des Gehäuses (14) vorgesehen ist, die die erste Kammer (24) von der zweiten Kammer (26) trennt.

5. Urinmessgerät (40) nach Anspruch 4, wobei das Rückschlagventil (42) ein Ventilelement (54), das an der inneren Trennwand (32a) montiert ist, und mindestens eine Öffnung (52) aufweist, die sich durch die innere Trennwand (32a) erstreckt und einen Fluidverbindungskanal zwischen der ersten und der zweiten Messkammer (24, 26) definiert.

6. Urinmessgerät (40) nach Anspruch 5, wobei das Ventilelement (54) einen Schaft (56) und einen Kopf (58) aufweist, wobei der Kopf (58) eine Dichtfläche (60) definiert, die zum Schließen der mindestens einen Öffnung (52) in Kontakt mit der inneren Trennwand (32a) bewegt werden kann.

7. Urinmessgerät (40) nach Anspruch 6, wobei die Dichtfläche (60) eine ringförmige Dichtfläche ist.

8. Urinmessgerät (40) nach Anspruch 6 oder 7, wobei der Schaft (56) des Ventilelements (54) in einer Öffnung (50) der inneren Trennwand (32a) aufgenommen ist.

9. Urinmessgerät (40) nach Anspruch 8, wobei das Ventilelement (54) durch den Kopf (58) des Ventilelements (54) und ein Gebilde (66) am Schaft (56) des Ventilelements (54) in der Öffnung (50) gehalten ist.

10. Urinmessgerät (40) nach Anspruch 9, wobei das Gebilde (66) am Schaft (56) des Ventilelements (54) federnd deformierbar ist, damit das Ventilelement (54) an die Öffnung (50) der inneren Trennwand (32a) angebracht werden kann.

11. Urinmessgerät (40) nach einem der Ansprüche 5 bis 10, wobei die innere Trennwand (32a) mit mehreren Öffnungen (52) versehen ist, die sich durch die innere Trennwand (32a) erstrecken und Fluidverbindungskanäle zwischen der ersten und zweiten Messkammer (24, 26) definieren.

12. Urinmessgerät (40) nach einem der vorhergehenden Ansprüche, wobei das Gehäuse (14) eine dritte Messkammer (28) aufweist, die im Gebrauch Flüssigkeit von der zweiten Messkammer (26) aufnimmt, wobei das Gehäuse (14) mit einem weiteren Rückschlagventil (42) zwischen der zweiten und der dritten Messkammer (26, 28) versehen ist, das im Gebrauch verhindert, dass Flüssigkeit von der dritten Messkammer (28) zu der zweiten Messkammer (26) fließt.

13. Urinmessgerät (40) nach Anspruch 12, wobei das zwischen der zweiten und der dritten Messkammer (26, 28) vorgesehene weitere Rückschlagventil (42) niedriger im Gehäuse (14) positioniert ist als das zwischen der ersten und der zweiten Messkammer (24, 26) vorgesehene Rückschlagventil (42).

## Revendications

1. Mesureur d'urine (40) ayant un boîtier (14) qui comprend une entrée (16) et une sortie (20), le boîtier (14) comprenant en outre une première chambre de mesure (24) et une deuxième chambre de mesure (26) agencées de sorte que le liquide entrant dans l'entrée (16) entre dans la première chambre de mesure (24) avant de passer dans la deuxième chambre de mesure (26), **caractérisé en ce que** le boîtier (14) est en outre prévu avec un clapet anti-retour (42) entre les première et deuxième chambres de mesure (24, 26) qui, à l'usage, empêche l'écoulement de liquide de la deuxième chambre de mesure (26) vers la première chambre de mesure (24).

2. Mesureur d'urine (40) selon la revendication 1, dans lequel le clapet anti-retour (42) est sollicité dans une condition ouverte pour permettre la communication de fluide entre la première chambre (24) et la deuxième chambre (26), et est poussé dans une condition fermée pour empêcher la communication de fluide entre les chambres (24, 26) par l'action du liquide présent à l'intérieur de la deuxième chambre (26).

3. Mesureur d'urine (40) selon la revendication 2, dans lequel le clapet anti-retour (42) est sollicité dans la condition ouverte par gravité.

4. Mesureur d'urine (40) selon l'une quelconque des revendications précédentes, dans lequel le clapet anti-retour (42) est prévu dans une séparation interne (32, 32a, 32b) du boîtier (14) qui sépare la première chambre (24) de la deuxième chambre (26).

5. Mesureur d'urine (40) selon la revendication 4, dans lequel le clapet anti-retour (42) comprend un élément de clapet (54) qui est monté dans la séparation interne (32a), et au moins une ouverture (52) qui s'étend à travers la séparation interne (32a) et qui définit un canal de communication de fluide entre les première et deuxième chambres de mesure (24, 26).

6. Mesureur d'urine (40) selon la revendication 5, dans lequel l'élément de clapet (54) comprend une tige (56) et une tête (58), dans lequel la tête (58) définit une surface d'étanchéité (60) qui est mobile en contact avec la séparation interne (32a) pour fermer la au moins une ouverture (52).

7. Mesureur d'urine (40) selon la revendication 6, dans lequel la surface d'étanchéité (60) est une surface d'étanchéité annulaire.

8. Mesureur d'urine (40) selon la revendication 6 ou 7, dans lequel la tige (56) de l'élément de clapet (54) est reçue dans une ouverture (50) de la séparation interne (32a).

9. Mesureur d'urine (40) selon la revendication 8, dans lequel l'élément de clapet (54) est retenu dans l'ouverture (50) de la séparation interne (32a) par la tête (58) de l'élément de clapet (54) et une formation (66) sur la tige (56) de l'élément de clapet (54).

10. Mesureur d'urine (40) selon la revendication 9, dans lequel la formation (66) sur la tige (56) de l'élément de clapet (54) est élastiquement déformable afin de permettre à l'élément de clapet (54) de s'adapter à l'ouverture (50) de la séparation interne (32a).

11. Mesureur d'urine (40) selon l'une quelconque des revendications 5 à 10, dans lequel la séparation interne (32a) est prévue avec une pluralité d'ouvertures (52) qui s'étendent à travers la séparation interne (32a) et qui définissent des canaux de communication de fluide entre les première et deuxième chambres de mesure (24, 26).

12. Mesureur d'urine (40) selon l'une quelconque des revendications précédentes, le boîtier (14) comprenant une troisième chambre de mesure (28) qui, à l'usage, reçoit le liquide de la deuxième chambre de mesure (26), dans lequel le boîtier (14) est prévu avec un autre clapet anti-retour (42) entre les deuxième et troisième chambres de mesure (26, 28) qui, à l'usage, empêche l'écoulement de liquide de la troisième chambre de mesure (28) à la deuxième chambre de mesure (26).

13. Mesureur d'urine (40) selon la revendication 12, dans lequel le clapet anti-retour supplémentaire (42) prévu entre les deuxième et troisième chambres de mesure (26, 28) est positionné plus bas dans le boîtier (14) que le clapet anti-retour (42) prévu entre les première et deuxième chambres de mesure (24, 26).
